# EUROPEAN PATENT SPECIFICATION

(11) **EP 1 486 514 B1**
(45) Date of publication and mention of the grant of the patent: **16.01.2008**
(21) Application number: 04013472.8
(22) Date of filing: 08.06.2004
(51) Int. Cl.: C08F 220/12

(54) **Acrylic polymer composition for cosmetics**
Harz für Kosmetika
Résine pour cosmétiques

(30) Priority: 11.06.2003 JP 2003166829
(43) Date of publication of application: 15.12.2004
(73) Proprietor: National Starch and Chemical Investment Holding Corporation, Wilmington, Delaware 19803-7663 (US)
(72) Inventor: Hashimoto, Tomohiro, Ikeda-Shi Osaka 563-0029 (JP); Ide, Hiroshi, Osaka 562-0025 (JP)
(74) Representative: Held, Stephan

(56) References cited:
- FR-A- 2 351 135
- US-A- 3 577 517

## Description

### BACKGROUND OF THE INVENTION

### 1. Field of the Invention

The present invention relates to a resin for cosmetics, a resin composition for cosmetics containing the resin as the main component and a cosmetic in which this resin composition is mixed. Particularly, the present invention relates to a cosmetic with excellent transparency that yields excellent balance between properties such as water resistance, adhesion, lasting effect and pulling feel, as well as to a resin for cosmetics for obtaining such cosmetic.

### DESCRIPTION OF THE PRIOR ART

For cosmetics such as, for example, hair cosmetics, make-up cosmetics, manicures and skin care cosmetics, water soluble polymer compounds have been mainly used as film-forming agents because of environmental and hygienic considerations. Water soluble polymers, however, have poor moisture resistance and water resistance and require a dissolving process in water when handled, so that they are time and labor consuming. For this reason, recently, polymer emulsions are widely used as aqueous film-forming agents, in place of water soluble polymers. In addition, polymer emulsions are advantageous in that they have moisture resistance and water resistance superior to those of water soluble polymers.

On the other hand, since a polymer emulsion is a suspension in which polymers are dispersed in water as fine particles, it is whitish in appearance. When applied onto skin or nails, this whitish suspension becomes dry and forms a transparent film. Accordingly, use of a polymer emulsion as the film-forming agent for a cosmetic can lead to a considerable difference between the color tone of the outward appearance of the cosmetic (before a film is formed) and the color tone of the cosmetic after use (the film formed as a result of drying). In order to eliminate the difference in these color tones, it is necessary to make the color tone of the outward appearance of the cosmetic (before a film is formed) transparent.
In this specification, "transparency" refers to a degree of the transparency of the color tone of the outward appearance of a cosmetic (before a film is formed), and, as mentioned below, is expressed by the transmittance of light having a wavelength of 425 nm that is measured in the state before a film is formed.

For the purpose of reducing the difference between the color tone of the outward appearance of a cosmetic and the color tone of the dried film, an aqueous manicure in which a polymer emulsion with a particle diameter of 0.1 µm or less is mixed is disclosed (see Patent Reference 1). However, a particle diameter of about 0.1 µm does not provide sufficient transparency. In order to obtain good transparency, it is necessary to reduce the particle diameter to about as small as 0.03 µm, and it is necessary to make the backbone of the polymer more hydrophilic in order to reduce the particle diameter to such degree. That is, when the polymer backbone is highly hydrophobic, the polymer is difficult to dissolve in water and thus easily becomes particulate. Therefore, it is believed that hydrophilization is necessary to avoid this. Hydrophilization of polymer backbones may present the problem that the water resistance of the resulting cosmetic is insufficient. Moreover, there is the problem that a polymer emulsion with a particle diameter of 0.1 µm or less may have a very high viscosity and may be difficult to be industrially produced.

On the other hand, Patent Reference 2 discloses an eyeliner excellent in water resistance, adhesion, writing feeling and the like, and Patent Reference 3 discloses a manicure. However, both of the references are silent about transparency. The transparency of the eyeliner of Patent Reference 2 is insufficient, and it seems that there is a significant difference between the color tones of the resin and the dried film of Patent Reference 2. As for the manicure of Patent Reference 3, the properties of the polymer serving as the film-forming agent are not clear. For example, it is unclear whether the polymer is a water soluble polymer or a polymer that forms an emulsion and whether it is a non-aqueous polymer obtained in an organic solvent.

As described above, a generally excellent resin for cosmetics serving as a film-forming agent for cosmetics that yields excellent balance between properties such as water resistance, adhesion, lasting effect and pulling feel despite having superior transparency has not been developed at present. It should be noted that "resin for cosmetics" in this specification refers to a polymer emulsion used as a film-forming agent for cosmetics.

Patent Reference 1
JP54-052736A
Patent Reference 2
JP55-130907A
Patent Reference 3
JP06-279239A

The present invention was achieved in order to solve the above-described problems, and it is an object of the present invention to provide a generally excellent resin for cosmetics serving as a film-forming agent for cosmetics that yields excellent balance between properties such as water resistance, adhesion, lasting effect and pulling feel despite having excellent transparency.

### SUMMARY OF THE INVENTION

The inventors found as the result of extensive studies that a film-forming agent that yields excellent balance between properties such as water resistance and adhesion can be obtained by improving the transparency by rendering the proportion of acids in a monomer mixture relatively high, as well as using both alkyl(meth)acrylates having a long chain alkyl group and alkyl(meth)acrylates having a short chain alkyl group as monomers at the time of synthesizing a resin for cosmetics and that this film-forming agent is generally excellent as a resin for cosmetics, and thus achieved the present invention.

According to one aspect of the present invention, a novel resin for cosmetics is provided, which is a resin for cosmetics obtained by emulsion polymerization of a monomer mixture containing:
(A) component: at least one selected from the group consisting of acrylic acid, methacrylic acid, maleic acid, itaconic acid and crotonic acid,
(B) component: at least one selected from the group consisting of alkyl(meth)acrylate monomers having an alkyl group with a carbon number of 1 to 4, and
(C) component: at least one selected from the group consisting of alkyl(meth)acrylate monomers having an alkyl group with a carbon number of 5 or more, wherein the monomer mixture contains 10 to 20 parts by weight of the (A) component, 40 to 80 parts by weight of the (B) component and 10 to 40 parts by weight of the (C) component, based upon the total of the (A) to (C) components (100 parts by weight).

By emulsion polymerization of a monomer mixture containing these (A) to (C) components at the ratio described above, it is possible to obtain a resin for cosmetics with superior transparency that yields excellent balance between properties such as water resistance, adhesion and lasting effect. Since this resin for cosmetics is synthesized by emulsion polymerization instead of by solution polymerization, it is desirable in terms of environmental and hygienic considerations as well as industrial productivity, and can be favorably used as a film-forming agent.

In one mode of the present invention, a resin for cosmetics is provided, wherein the (A) component is methacrylic acid.
In another mode of the present invention, a resin for cosmetics is provided, wherein a (D) reactive emulsifier is used at the time of the emulsion polymerization and the (D) reactive emulsifier is an anionic surfactant and/or nonionic surfactant.
In a preferred mode of the present invention, a resin for cosmetics is provided, which has a transmittance of light with a wavelength of 425 nm of at least 50%.
In another preferred mode of the present invention, a resin for cosmetics is provided, wherein a (E) chain transfer agent is further used at the time of the emulsion polymerization.

In another aspect of the present invention, a resin composition for cosmetics is provided, which contains the above-described resin for cosmetics as a main component.
In a preferred aspect of the present invention, a cosmetic is provided, in which the above-described resin composition for cosmetics is mixed as one component.

### DESCRIPTION OF THE PREFERRED EMBODIMENTS

In the present invention, "(A) component" is a component that serves as an acid, and refers to at least one selected from the group consisting of acrylic acid, methacrylic acid, maleic acid, itaconic acid and crotonic acid. Acrylic acid, methacrylic acid, maleic acid, itaconic acid and crotonic acid may be used alone or in combination. As the (A) component, it is particularly preferable to use methacrylic acid.

In the present invention, "(B) component" refers to at least one selected from the group consisting of alkyl(meth)acrylate monomers having an alkyl group with a carbon number of 1 to 4, and "alkyl(meth)acrylate monomers having an alkyl group with a carbon number of 1 to 4" refer to acrylate monomers having an alkyl group with a carbon number of 1 to 4 and methacrylate monomers having an alkyl group with a carbon number of 1 to 4. These may be used alone or in combination.

Specific examples of "alkyl(meth)acrylate monomers having an alkyl group with a carbon number of 1 to 4" include methyl acrylate, methyl methacrylate, ethyl acrylate, ethyl methacrylate, propyl acrylate, propyl methacrylate, butyl acrylate and butyl methacrylate.

"(C) component" of the present invention refers to at least one selected from the group consisting of meth(acrylate) monomers having an alkyl group with a carbon number of 5 or more, and "meth(acrylate) monomers having an alkyl group with a carbon number of 5 or more" refer to acrylate monomers having an alkyl group with a carbon number of 5 or more and methacrylate monomers having an alkyl group with a carbon number of 5 or more. These may be used alone or in combination.

Specific examples of "meth(acrylate) monomers having an alkyl group with a carbon number of 5 or more" include hexyl acrylate, hexyl methacrylate, 2-ethylhexyl-acrylate, 2-ethylhexyl-methacrylate, octyl acrylate, octyl methacrylate, lauryl acrylate and lauryl methacrylate.

A resin for cosmetics of the present invention can be obtained by the emulsion polymerization of a monomer mixture containing the above-described (A) to (C) components, and the monomer mixture contains 10 to 20 parts by weight of the (A) component, 40 to 80 parts by weight of the (B) component and 10 to 40 parts by weight of the (C) component, based upon the total of the (A) to (C) components (100 parts by weight). The monomer mixture preferably contains 10 to 17 parts by weight of the (A) component, 50 to 80 parts by weight of the (B) component and 10 to 35 parts by weight of the (C) component, and more preferably 10 to 15 parts by weight of the (A) component, 60 to 80 parts by weight of the (B) component and 10 to 30 parts by weight of the (C) component.

When the amount of the (A) to (C) components contained in the monomer mixture are out of the ranges of 10 to 20 parts by weight for the (A) component, 40 to 80 parts by weight for the (B) component and 10 to 40 parts by weight for the (C) component, there may be the problem that it is impossible to obtain a cosmetic with superior transparency that yields excellent balance between properties such as water resistance, adhesion, lasting effect and pulling feel, and a resin for cosmetics for obtaining such cosmetic.

When the (A) component is less than 10 parts by weight, the balance between the properties of transparency, adhesion, lasting effect and pulling feel deteriorates. When the (A) component is more than 20 parts by weight, the balance between the properties of water resistance, lasting effect and pulling feel deteriorates.
When the (B) component is less than 40 parts by weight, the balance between the properties of water resistance, lasting effect and pulling feel deteriorates. When the (B) component is more than 80 parts by weight, the balance between the properties of transparency, adhesion, lasting effect and pulling feel deteriorates.
When the (C) component is less than 10 parts by weight, the balance between the properties of water resistance, lasting effect and pulling feel deteriorates. When the (C) component is more than 40 parts by weight, the balance between the properties of transparency, adhesion, lasting effect and pulling feel deteriorates.

Furthermore, the above-described monomer mixture may contain another monomer (M) having an ethylenic double bond that is copolymerizable with the components (A) to (C). Examples of such "another monomer (M)" include styrene, hydroxyethyl acrylate, hydroxyethyl methacrylate, hydroxypropyl acrylate and hydroxypropyl methacrylate.
The monomer mixture preferably contains 0 to 20 parts by weight of another monomer (M), more preferably 0 to 15 parts by weight, and most preferably 0 to 10 parts by weight, based upon the total of the (A) to (C) components (100 parts by weight).

A resin for cosmetics of the present invention can be obtained by the emulsion polymerization of the above-described monomer mixture containing the (A) to (C) components (in some cases, a monomer mixture further containing the (M) component). As the emulsion polymerization, any common emulsion polymerization process can be used as long as the present invention can obtain a target resin for cosmetics.

When performing the emulsion polymerization of a monomer mixture, an aqueous medium is used as a polymerization reaction medium. "Aqueous medium" may be water or a "mixed solvent of a water soluble solvent and water", and there is no particular limitation as long as it does not adversely affect the resulting resin for cosmetics. Examples of "water" mentioned herein include running tap water, distilled water and ion-exchange water.

Examples of "water soluble solvent" include the following solvents:
lower alcohols such as methanol, ethanol and isopanol;
ketones such as acetone, methyl ethyl ketone and methyl butyl ketone;
ketone alcohols such as diacetone alcohol;
ethers such as tetrahydrofuran and dioxane;
esters such as ethyl acetate; and
amides such as dimethylformamide and dimethylacetamide.
"Water" and "water soluble solvent" described herein may be used alone or in combination.

Additionally, it is preferable to use an emulsifier at the time of the emulsion polymerization.
"Emulsifier" may be any emulsifier that can be ordinarily used as an emulsifier, and there is no particular limitation. Examples include anionic surfactants, nonionic surfactants and cationic surfactants.

"Anionic surfactants" refer to surfactants that can be ionized in aqueous solutions and whose hydrophilic portions are anions. "Nonionic surfactants" refer to surfactants that exhibit surface activity without dissociating into ions in aqueous solutions. In addition, "cationic surfactants" refer to surfactants that can be ionized in aqueous solutions and whose hydrophilic portions are cations.

Examples of "anionic surfactants" include higher fatty acid salts, alkylaryl sulfonate, alkyl sulfate, polyoxyethylene alkyl sulfate, polyoxyethylene alkyl ether sulfate and alkyl sulfonate.
Examples of "nonionic surfactants" include polyoxyethylene alkyl ether, polyoxyethylene oxypropylene alkyl ether, polyoxyethylene alkyl phenol ether, sorbitan higher fatty acid ester, polyoxyalkyl aryl ether and oxyethylene oxypropylene block copolymer.
The emulsifier is preferably at least one selected from the group consisting of anionic surfactants and nonionic surfactants. The emulsifier may be used alone or in combination.

More preferably, the emulsifier is a (D) reactive emulsifier. "(D) reactive emulsifier" herein refers to compounds that have a polymerizable ethylenic unsaturated bond and can serve as emulsifiers for forming emulsions. Use of "(D) reactive emulsifier" can provide a polymer emulsion that yields better balance between properties such as transparency, water resistance, adhesion, lasting effect and pulling feel, as compared to use of a common emulsifier.

"(D) reactive emulsifier" can also be classified into anionic surfactants, nonionic surfactants and cationic surfactants, depending on their hydrophilic groups. As "(D) reactive emulsifier", anionic surfactants and nonionic surfactants are preferable.
Examples of the (D) reactive emulsifier include anionic surfactants such as monomers that have a carboxyl group, a sulfonic acid group, a sulfonate group or a sulfate group (these functional groups include, for example, functional groups in the forms of their salts such as a carboxylic acid base and a sulfonic acid base) and additionally have an ethylenic double bond, as well as nonionic surfactants such as monomers that have an ethylenoxy group and additionally have an ethylenic double bond.

The (D) reactive emulsifier is preferably an anionic surfactant, and more preferably an anionic surfactant having a sulfonic acid group or a sulfonate group. Particularly, a sulfonic acid group and a sulfonate group are preferably in the form of salts. Further, the countercation of a sulfonic acid group or a sulfonate group is preferably an ammonium ion and an alkali metal ion. As the countercation, an ammonium ion, a potassium ion and a sodium ion are particularly preferable.

Examples of such "(D) reactive emulsifier" include ELEMINOL JS-2 (trademark) manufactured by Sanyo Chemical Industries, Ltd, which is an anionic surfactant, and PE350 (trademark) manufactured by NOF CORPORATION, which is a nonionic surfactant. These reactive emulsifiers may be used alone or in combination.

The emulsifier is preferably used in an amount of 0.05 to 10 parts by weight, more preferably 0.1 to 7 parts by weight, and most preferably 0.1 to 5 parts by weight, based upon the total amount (100 parts by weight) of the (A) to (C) components (or based upon the total amount (100 parts by weight) of the (A) to (C) and (M) components, if the monomer mixture contains the (M) component).

The (D) reactive emulsifier is preferably used in an amount of 0.05 to 10 parts by weight, more preferably 0.1 to 5 parts by weight, and most preferably 0.1 to 2 parts by weight, based upon the total amount (100 parts by weight) of the (A) to (C) components (or based upon the total amount (100 parts by weight) of the (A) to (C) and (M) components, if where the monomer mixture contains the (M) component).

In addition, use of "cationic surfactant" as the emulsifier for emulsion polymerization may reduce the degree of the polymerization of the (A) component, and thus possibly making it difficult to obtain a polymer emulsion that yields excellent balance between transparency and water resistance. This is presumably because the above-described monomer mixture may contain a relatively large amount of the (A) component, which is an acid, and use of the cationic surfactant may result in the formation of a complex between this (A) component and the cationic surfactant.

Additionally, it is also preferable to use a (E) chain transfer agent for the emulsion polymerization of the above-described monomer mixture. "(E) chain transfer agent" refers to compounds that can control the molecular weight of a target resin for cosmetics by a small addition.
Specific examples of "(E) chain transfer agent" include thiol compounds such as 2-mercaptoethanol, n-octyl mercaptan and n-dodecyl mercaptan; disulfides such as tetramethylthiuramdisulfide, and halogen compounds such as chloroform.
Particularly, n-dodecyl mercaptan is preferable. The (E) chain transfer agent may be used alone or in combination.

The (E) chain transfer agent is preferably used in an amount of 0.1 to 5 parts by weight, more preferably 0.1 to 4 parts by weight, and most preferably 0.1 to 2 parts by weight, based upon the total amount (100 parts by weight) of the (A) to (C) components (or based upon the total amount (100 parts by weight) of the (A) to (C) and (M) components, if where the monomer mixture contains the (M) component).

Preferably, the emulsion polymerization is initiated by using a polymerization initiator. As such "polymerization initiator", it is possible to use compounds that can initiate the polymerization reaction of the monomer mixture containing the (A) to (C) components by a small addition and that can be used in an aqueous medium.

Examples of such polymerization initiator include the following compounds:
persulfates such as ammonium persulfate, sodium persulfate and potassium persulfate;
organic peroxides such as t-butyl hydroperoxide and t-butyl peroxybenzoate;
azo compounds such as 2,2-azobisisobutyronitrile (AIBN), 2,2-azobis(2-diamidinopropane)dehydrochloride and 2,2-azobis(2,4-dimethylvaleronitrile); and
inorganic peroxides such as hydrogen peroxide.
As the polymerization initiator, persulfates such as ammonium persulfate, sodium persulfate and potassium persulfate are particularly preferable.
The polymerization initiator may be used alone or in combination.

When the pH of a resin for cosmetics obtained by the emulsion polymerization is outside the neutral range, it is preferable to neutralize the pH with a neutralizing agent. As the neutralizing agent, it is possible to employ commonly used neutralizing agents, for example, amine aqueous solutions such as ammonia water, triethanolamine and 2-amino-2-methyl-1-propanol, and solutions of metal hydroxides such as potassium hydroxide and sodium hydroxide.

An example of the process for producing a resin for cosmetics of the present invention is described below.
A monomer mixture containing the above-described (A) to (C) components at the ratio described above is charged into an aqueous medium, and the mixture in which the (A) to (C) components are dissolved and/or dispersed in the aqueous medium is heated to a predetermined temperature. Thereafter, a polymerization initiator and an emulsifier are added to this mixture to perform the emulsion polymerization of the (A) to (C) components. The polymerization initiator and the emulsifier may also be added to the aqueous medium simultaneously when the monomer mixture is charged into the aqueous medium. Additionally, the (E) chain transfer agent may be added to this mixture. After heating the mixture at a predetermined temperature, a resin for cosmetics of the present invention can be obtained in the form of an emulsion in which polymer particles obtained by the polymerization are dispersed in the aqueous medium. As necessary, a neutralizing agent may be added to this emulsion to obtain a resin for cosmetics of the present invention.
As described above, the (D) reactive emulsifier is particularly preferable as the emulsifier.

The weight average molecular weight of a resin for cosmetics of the present invention obtained as described above is preferably 1000 to 100000. The weight average molecular weight is more preferably 5000 to 80000, and most preferably 10000 to 60000. When the weight average molecular weight of the resin for cosmetics is less than 1000, the balance between the properties of water resistance, adhesion and lasting effect may be insufficient. On the other hand, when the weight average molecular weight of the resin for cosmetics is more than 100000, the balance between the properties of transparency and pulling feel may be insufficient and the viscosity may be high, so that it may be difficult to mix the resin for cosmetics with other additives.

In this specification, "weight average molecular weight" refers to a weight average molecular weight in terms of polyethylene glycol measured by using gel permeation chromatography (hereinafter referred to as "GPC"). The details are described in the examples.

"Transparency" of a resin for cosmetics of the present invention refers to a degree of transparency of the color tone of the outward appearance of the cosmetic (before a film is formed). More specifically, "degree of transparency" can be evaluated by the transmittance of light (wavelength: 425 nm) corresponding to the state before a film is formed. "Transmittance" of light can be measured with a spectrophotometer.
That is, "transmittance" of a resin for cosmetics of the present invention is at least 50% in an aqueous solution with a resin content of 0.6 wt%, relative to the transmittance of the aqueous medium before it contains resin taken as 100%. More preferably, the transmittance is 55% or more, and most preferably 60% or more. When the transmittance is less than 50%, the difference in color tones between the outward appearance of a cosmetic obtained by the resin for cosmetics and a dried film formed therefrom may become too great.
In this specification, "resin content" refers to a resin solid content.

Ordinarily, a resin for cosmetics of the present invention takes the form of an emulsion containing a resin obtained by emulsion polymerization and an aqueous medium. It is also possible to use only a resin obtained by emulsion polymerization after separating the resin obtained by emulsion polymerization and an aqueous medium. Thereafter, an emulsion may be formed by adding the aqueous medium to the resin obtained by emulsion polymerization. In the present invention, "parts by weight" and "wt%" are based upon the weight excluding the aqueous medium, unless otherwise described.

A resin composition for cosmetics can be obtained by mixing various additives to the thus obtained resin for cosmetics. As described above, "resin composition for cosmetics" of the present invention refers to a resin composition in which a resin for cosmetics as the main component and various additives are mixed. The mixing method may be a common method, and any known method commonly used for mixing may be employed.

Examples of additives that are mixed with "resin for cosmetics" include water, lower alcohols such as ethanol, nonionic surfactants, anionic surfactants, cationic surfactants, amphoteric surfactants, amino acid-based surfactants, fatty acid soaps, oils and fats, waxes, hydrocarbon oils, ester oils, higher alcohols, polyhydric alcohols, silicons (or silicones), polymer compounds, viscosity increasing agents, powders, ultraviolet absorbers, moisturizing agents, antiseptics, antibacterial agents, aroma chemicals, antioxidants, pH modifiers, chelating agents, refrigerants, antiinflammatory agents and skin care components (e.g., skin-lightening agents, cell activators, skin roughness inhibitors and blood circulation promoters).
These additives may be used alone or in combination.

"Anionic surfactants" as the additives that are mixed with "resin for cosmetics" refer to any anionic surfactants that are commonly used for mixing with "resin for cosmetics", and there is no particular limitation. It should be noted that the (D) reactive emulsifier used for the emulsion polymerization of a monomer mixture containing the (A) to (C) components are not included among these surfactants.
Examples of "anionic surfactants" as additives that are mixed with "resin for cosmetics" include the following compounds:
polyoxyethylene alkyl ether sulfates such as sodium polyoxyethylene lauryl ether sulfate and ammonium polyoxyethylene lauryl ether sulfate;
alkyl sulfates such as sodium lauryl sulfate and ammonium lauryl sulfate;
sodium polyoxyethylene alkyl ether acetate such as sodium polyoxyethylene tridecyl ether acetate and sodium polyoxyethylene lauryl ether acetate;
alkyl sulfosuccinates such as dialkylsulfosuccinic acid, polyoxyalkylene alkyl sulfosuccinate and dioctyl sodium sulfosuccinate;
polyoxyethylene fatty acid amide ether sulfate, sodium cocoyl methyl taurate, N-acyl-L-aspartate, cocoyl ethyl ester sulfonate, sodium alkylβ-alanine, acylmethyl taurine, alkyl ethane sodium sulfonate, sodium polyoxyethylene alkyl ether carboxylate, alkane sulfonate, olefin sulfonate and alkyl benzene sulfonate.
The anionic surfactants may be used alone or in combination.

"Nonionic surfactants" as additives that are mixed with "resin for cosmetics" refer to any nonionic surfactants that are commonly used for mixing with "resin for cosmetics", and there is no particular limitation. It should be noted that the (D) reactive emulsifier used for the emulsion polymerization of a monomer mixture containing the (A) to (C) components are not included among these surfactants.
Examples of "nonionic surfactants" as additives that are mixed with "resin for cosmetics" include the following compounds:
polyoxyethylene alkyl ethers such as polyoxyethylene lauryl ether, polyoxyethylene cetyl ether, polyoxyethylene stearyl ether, polyoxyethylene oleyl ether and polyoxyethylene behenyl ether;
polyoxyethylene polyoxypropylene alkyl ethers such as polyoxyethylene polyoxypropylene cetyl ether and polyoxyethylene polyoxypropylene decyltetradecyl ether;
diethanolamides such as coconut oil fatty acid diethanolamide, lauric acid diethanolamide, myristic acid diethanolamide, palmitic acid diethanolamide, stearic acid diethanolamide, isostearic acid diethanolamide and oleic acid diethanolamide;
monoethanolamides such as coconut oil fatty acid monoethanolamide, lauric acid monoethanolamide, myristic acid monoethanolamide, palmitic acid monoethanolamide, stearic acid monoethanolamide, isostearic acid monoethanolamide and oleic acid monoethanolamide;
isopropanolamides such as coconut oil fatty acid isopropanolamide, lauric acid isopropanolamide, myristic acid isopropanolamide, palmitic acid isopropanolamide, stearic acid isopropanolamide, isostearic acid isopropanolamide and oleic acid isopropanolamide;
polyoxyethylene monoethanolamides such as polyoxyethylene coconut oil fatty acid monoethanolamide, polyoxyethylene lauric acid monoethanolamide, polyoxyethylene myristic acid monoethanolamide, polyoxyethylene palmitic acid monoethanolamide, polyoxyethylene stearic acid monoethanolamide, polyoxyethylene isostearic acid monoethanolamide and polyoxyethylene oleic acid monoethanolamide;
alkyl glucosides such as decyl glucoside and lauryl glucoside;
alkyl dimethylamine oxides such as lauryl dimethylamine oxide and stearyl dimethylamine oxide;
hydroxyl fatty acid alkyl maltitol ether, alkylated polysaccharides, saccharose fatty acid ester and fatty acid isopropanolamide.
The nonionic surfactants may be used alone or in combination.

"Cationic surfactants" as additives that are mixed with "resin for cosmetics" refer to any cationic surfactants that are commonly used for mixing with "resin for cosmetics", and there is no particular limitation. It should be noted that the (D) reactive emulsifier used for the emulsion polymerization of a monomer mixture containing the (A) to (C) components are not included among these surfactants.
Examples of "cationic surfactants" as additives that are mixed with "resin for cosmetics" include the following compounds:
alkyl trimethyl ammonium chlorides such as lauryl trimethyl ammonium chloride, cetyl trimethyl ammonium chloride, stearyl trimethyl ammonium chloride and behenyl trimethyl ammonium chloride;
alkyl dimethyl ammonium chlorides such as distearyl dimethyl ammonium chloride and dialkyl (C12 to 18) dimethyl ammonium chloride;
lanolin derivatives of quaternary ammonium salts, behenyl dimethyl hydroxyethyl ammonium chloride and stearyl dimethyl benzyl ammonium chloride.
The cationic surfactants may be used alone or in combination.

"Amphoteric surfactants" as additives that are mixed with "resin for cosmetics" refer to any amphoteric surfactants that are commonly used for mixing with "resin for cosmetics", and there is no particular limitation. It should be noted that the (D) reactive emulsifier used for the emulsion polymerization of a monomer mixture containing the (A) to (C) components are not included among these surfactants.
Examples of "amphoteric surfactants" as additives that are mixed with "resin for cosmetics" include the following compounds:
acetic acid betaines such as lauryldimethylaminoacetic acid betaine, trialkylaminoacetic acid betaine and stearyldimethylaminoacetic acid betaine;
amidopropylbetaines such as lauric acid amidopropylbetaine, coconut oil fatty acid amidopropylbetaine, myristic acid amidopropylbetaine and cocoalkyl-N-carboxyethyl-N-hydroxyethyl imidazolinium betaine; and
imidazolinium betaines such as alkyl-N-hydroxyethyl-N-carboxymethylimidazolinium betaine and 2-alkyl-N-carboxymethyl-N-hydroxyethylimidazoliinum betaine.
The amphoteric surfactants may be used alone or in combination.

"Amino acid-based surfactants" as additives that are mixed with "resin for cosmetics" refer to any amino acid-based surfactants that are commonly used for mixing with "resin for cosmetics", and there is no particular limitation. It should be noted that the (D) reactive emulsifier used for the emulsion polymerization of a monomer mixture containing the (A) to (C) components are not included among these surfactants.
Examples of "amino acid-based surfactants" as additives that are mixed with "resin for cosmetics" include the following compounds:
glutamates such as triethanolamine salts, sodium salts and potassium salts of N-coconut oil fatty acid acyl-L-glutamic acid, lauroyl-L-glutamic acid, myristoyl-L-glutamic acid, stearoyl-L-glutamic acid and the like;
glycine salts such as triethanolamine salts, sodium salts and potassium salts of N-coconut oil fatty acid acyl glycine, lauroyl glycine, myristoyl glycine, stearoyl glycine and the like;
alanine salts such as triethanolamine salts, sodium salts and potassium salts of N-coconut oil fatty acid acyl alanine, lauroyl alanine, myristoyl alanine, stearoyl alanine and the like;
sarcosine salts such as triethanolamine salts, sodium salts and potassium salts of N-coconut oil fatty acid acyl sarcosine, lauroyl sarcosine, myristoyl sarcosine, stearoyl sarcosine and the like;
N-coconut oil fatty acid acyl-L-arginine ethyl DL-pyrrolidone carboxylate, N-acyl taurate and N-acyl methyltaurine.
The amino acid-based surfactants may be used alone or in combination.

"Fatty acid soaps" refer to any fatty acid soaps that are commonly used for mixing with "resin for cosmetics", and there is no particular limitation. Examples of fatty acid soaps include alkali salts of C6-24 higher fatty acids. The fatty acids may be either saturated or unsaturated, and examples include capric acid, lauric acid, myristic acid, palmitic acid, stearic acid, isostearic acid, arachidic acid, behenic acid, oleic acid, isooleic acid, linoleic acid, linolenic acid and arachidonic acid. In addition, the alkalis for neutralizing the fatty acids may be any alkalis that are commonly used for producing soaps, and there is no particular limitation. Examples include caustic potash, caustic soda, triethanolamine, N-methyltaurine and ammonia.
The fatty acid soaps may be used alone or in combination.

"Oils and fats" may be any oils and fats that are commonly used for mixing with the resin for cosmetics, and there is no particular limitation. Examples of oils and fats include avocado oil, camellia oil, evening primrose oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, China wood oil, Japanese wood oil, jojoba oil, germ oil, triglycerol, glyceryl trioctanoate, glycerol triisopalmitate, cacao butter, coconut oil, hardened coconut oil, palm oil, palm kernel oil, Japan wax kernel oil, hardened oil, hardened castor oil and their polyoxyethylene adducts.
The fats and oils may be used alone or in combination.

"Waxes" refer to any waxes that are commonly used for mixing with the resin for cosmetics, and there is no particular limitation. Examples of waxes include spermaceti, beeswax, high acid number beeswax, shellac, mink wax, lanolin, lanolin acetate, liquid lanolin, carnauba wax, candelilla wax, rice bran wax, bran wax, Japan wax, cotton wax, bayberry wax, Chinese insect wax (Ibota wax) , montan wax, kapok wax, jojoba wax, sugarcane wax, lanolin fatty acid isopropyl, hexyl laurate, reduced lanolin, hard lanolin, shellac wax, POE lanolin alcohol ether, POE lanolin alcohol acetate, POE cholesterol ether, lanolin fatty acid polyethylene glycol and POE hydrogenated lanolin alcohol ether.
The waxes may be used alone or in combination.

"Hydrocarbon oils" refer to any hydrocarbon oils that are commonly used for mixing with the resin for cosmetics, and there is no particular limitation.
Examples of hydrocarbon oils include oil components of paraffin, liquid paraffin, ozokerite, squalene, pristine, ceresin, vaseline and microcrystalline wax.
The hydrocarbon oils may be used alone or in combination.

"Ester oils" refer to any ester oils that are commonly used for mixing with the resin for cosmetics, and there is no particular limitation.
Examples of ester oils include the following compounds:
myristates such as isopropyl myristate, butyl myristate, myristyl myristate, isocetyl myristate, octyldodecyl myristate and 2-hexyldecyl myristate;
palmitates such as isopropyl palmitate, cetyl palmitate, isostearyl palmitate, 2-ethylhexyl palmitate, 2-hexyldecyl palmitate and 2-heptylundecyl palmitate;
stearates such as butyl stearate, isocetyl stearate, cholesteryl stearate, isocetyl isostearate, cholesteryl 12-hydroxystearate and N-alkylglycol isostearate;
laurates such as isopropyl laurate and hexyl laurate;
linoleates such as ethyl linoleate and isopropyl linoleate;
octanoates such as cetyl octanoate, hexyldecyl dimethyloctanoate, octyldodecyl dimethyloctanoate and cetyl isooctanoate;
oleates such as decyl oleate and oleic acid oil;
sorbitan fatty acid ester oils such as sorbitan monolaurate, sorbitan monopalmitate, sorbitan monostearate, sorbitan sesquistearate, sorbitan tristearate, sorbitan monooleate, sorbitan sesquioleate, sorbitan trioleate, sorbitan monoisostearate and sorbitan sesquiisostearate;
polyoxyethylene sorbitan fatty acid esters such as polyoxyethylene sorbitan monococoate, polyoxyethylene sorbitan monopalmitate, polyoxyethylene sorbitan monostearate, polyoxyethylene sorbitan monoisostearate, polyoxyethylene sorbitan tristearate and polyoxyethylene sorbitan monooleate;
polyoxyethylene sorbitol fatty acid esters such as polyoxyethylene sorbitol monolaurate, polyoxyethylene sorbitol hexastearate, polyoxyethylene sorbitol tetrastearate and polyoxyethylene sorbitol tetraoleate;
lactates such as cetyl lactate and myristyl lactate;
malates such as diisostearyl malate;
adipates such as diisobutyl adipate, di-2-heptylundecyl adipate and 2-hexyldecyl adipate;
sebacates such as di-2-ethylhexyl sebacate and diisopropyl sebacate;
succinates such as 2-ethylhexyl succinate;
citrates such as triethyl citrate, triisocetyl citrate, triisoarachyl citrate, triisooctyl citrate, acetyltriethyl citrate and acetyltributyl citrate;
polyhydric alcohol esters such as ethylene glycol di-2-ethylhexanoate, propylene glycol monocaprate, propylene glycol dicaprate, propylene glycol didecanoate, glyceryl tri-2-ethylhexanoate, glyceryl tri(caprylate/caprate), trimethylolpropane tri-2-ethylhexanoate, trimethylolpropane triisostearate, pentaerythritol tetra-2-ethylhexanoate, glyceryl trimyristate, decaglyceryl decastearate, decaglyceryl decaoleate, decaglyceryl decaisostearate, glyceryl di-2-heptylundecanoate, polyoxyethylene glyceryl monostearate, polyethylene glycol glyceryl monooleate, diglyceryl monostearate, diglyceryl monoisostearate, diglyceryl monooleate, tetraglyceryl monostearate, polyglyceryl monooleate, polyglyceryl tristearate, polyglyceryl pentastearate, polyglyceryl pentaoleate and neopentyl glycol dicaprate;
lanolin acetate, dipentaerythritol fatty acid ester, glyceride tri-2-heptylundecanoate, castor oil fatty acid methyl ester, acetoglyceride, N-lauroyl-L-glutamic acid-2-octyldodecyl ester and ethyl laurate.
The ester oils may be used alone or in combination.

"Higher alcohols" refer to any higher alcohols that are commonly used for mixing with the resin for cosmetics, and there is no particular limitation. Examples of higher alcohols include lauryl alcohol, cetyl alcohol, stearyl alcohol, cetostearyl alcohol, cetearyl alcohol, behenyl alcohol, myristyl alcohol, oleyl alcohol, batyl alcohol, capryl alcohol, 2-decyltetradecinol, lanolin alcohol, cholesterol, phytosterol, hexyl dodecanol, isostearyl alcohol and octyl dodecanol.
The higher alcohols may be used alone or in combination.

"Polyhydric alcohols" refer to any polyhydric alcohols that are commonly used for mixing with the resin for cosmetics, and there is no particular limitation. Examples of polyhydric alcohols include 1,2-propanediol, 1,3-propanediol, 1,2-butanediol, 1,3-butanediol, 1,4-butanediol, dipropylene glycol, glycerol, diglycerol, sorbitan, sorbitol and maltitol.
The polyhydric alcohols may be used alone or in combination.

"Silicons" refer to any silicons that are commonly used for mixing with the resin for cosmetics, and there is no particular limitation. Examples of silicons include amino modified silicons such as amodimethicone, polyether modified silicons such as dimethicone polyol, cyclic silicons such as cyclomethicone, dimethicones such as methyl polysiloxane and highly polymerized methyl polysiloxane, phenyl modified silicons such as methylphenyl polysiloxane, and alkyl modified silicons and alkoxy modified silicons.
The silicons may be used alone or in combination.

"Powders" refer to any powders that are commonly used for mixing with the resin for cosmetics, and there is no particular limitation. Powders can be used regardless of their shapes (e.g., spherical, needle-shaped and plate-shaped), particle sizes (e.g., fume form, and fine particle and pigment grades), particle structures (e.g., porous and nonporous) and the like.
Examples of "inorganic powders" include titanium oxide, zirconium oxide, zinc oxide, cerium oxide, magnesium oxide, barium sulfate, calcium sulfate, magnesium sulfate, calcium carbonate, magnesium carbonate, talcs, synthetic micas, micas, kaolin, sericite, muscovite, phlogopite, lepidolite, biotite, lithia mica, silicic acid, silicic acid anhydride, aluminum silicate, magnesium silicate, aluminum magnesium silicate, calcium silicate, barium silicate, strontium silicate, metal salts of tungstic acid, hydroxyapatite, vermiculite, haidilite, bentonite, montmorillonite, hectorite, zeolite, ceramics powder, diabasic calcium phosphate, alumina, aluminum hydroxide, boron nitride and silica.

Examples of "organic powders" include starch powders, polyamide powder, polyester powder, polyethylene powder, polypropylene powder, polystyrene powder, polyurethane powder, benzoguanamine powder, polymethylbenzoguanamine powder, tetrafluoroethylene powder, polymethyl methacrylate powder, celluloses, silk powder, nylon powder, Nylon 12, Nylon 6, styrene-acrylic acid copolymers, divinylbenzene-styrene copolymers, vinyl resins, urea resins, phenolic resins, fluororesins, silicon resins, acrylic resins, melamine resins, epoxy resins, polycarbonate resins, microcrystalline fiber powder, rice starch and lauroyl lysine.
The powders may be used alone or in combination.

"Polymer compounds" refer to any polymer compounds that are commonly used for mixing with the resin for cosmetics, and there is no particular limitation. The polymer compounds can be classified into natural polymer compounds, semi-synthetic polymer compounds and synthetic polymer compounds.
Examples of "natural polymers" include gum arabic, tragacanth gum, galactan, guar gum, carob gum, karaya gum, carrageenan, pectin, agar, quince seed (marmelo), algae colloid (algae extract), starches (rice, corn, potato, wheat), glycyrrhizin, xanthan gum, dehydroxanthan gum, dextran, succinoglucan, pullulan, collagen, casein, albumin and gelatin.

Examples of "semi-synthetic polymer compounds" include starch compounds such as carboxymethyl starches, methylhydroxypropyl starches, modified potato starches, modified corn starches and hydroxypropyl starch phosphate; cellulose polymers such as methylcellulose, nitrocellulose, ethylcellulose, hydroxypropyl methylcellulose, hydroxyethylcellulose, sodium cellulose sulfate, hydroxypropyl cellulose, sodium carboxymethylcellulose (CMC), crystalline cellulose and cellulose powders; and alginic acid polymers such as sodium alginate and propylene glycol alginate.

Examples of synthetic polymer compounds include: vinyl based polymers such as polyvinyl alcohol, polyvinyl methyl ether, polyvinyl pyrrolidone and carboxyvinyl polymers (Carboball); polyoxyethylene polymers such as polyethylene glycols 2000, 4000 and 6000; polyoxyethylene polyoxypropylene copolymer polymers; vinyl acetate polymers such as VA/crotonic acid/neodecanoic acid vinyl copolymers; acryl polymers such as sodium polyacrylate, octylacrylamide/hydroxypropyl acrylate/butylaminoethyl methacrylate copolymers, alkyl acrylate/octylacrylamide copolymers, poly(metha)crylates and polyacrylamides; and polyethylene imine and cation polymers.
These polymer compounds may be in any form, including, for example, the form of solution, fine particles (dispersion) or particles (emulsion).
The polymer compounds may be used alone or in combination.

"Ultraviolet absorbers" refer to any ultraviolet absorbers that are commonly used for mixing with the resin for cosmetics, and there is no particular limitation.
Examples of ultraviolet absorbers include the following compounds:
benzophenone derivatives such as 2-hydroxy-4-methoxybenzophenone, 2-hydroxy-4-methoxybenzophenone-5-sulfonic acid, 2-hydroxy-4-methoxybenzophenone-5-sodium sulfonate, dihydroxymethoxybenzophenone, dihydroxymethoxybenzophenone-sodium sulfonate, 2,4-dihydroxybenzophenone and tetrahydroxybenzophenone;
*p*-aminobenzoic acid derivatives such as *p*-aminobenzoic acid, ethyl *p*-aminobenzoate, glyceryl *p*-aminobenzoate, amyl *p*-dimethylaminobenzoate and octyl p-dimethylaminobenzoate;
methoxycinnamic acid derivatives such as ethyl *p*-methoxycinnamate, isopropyl p-methoxycinnamate, octyl *p*-methoxycinnamate, 2-ethoxyethyl *p*-methoxycinnamate, sodium *p*-methoxycinnamate, potassium *p*-methoxycinnamate and glyceryl mono-2-ethylhexanoate *p*-metoxy cinnamate;
salicylic acid derivatives such as octyl salicylate, phenyl salicylate, homomenthyl salicylate, dipropylene glycol salicylate, ethylene glycol salicylate, myristyl salicylate and methyl salicylate;
urocanic acid, ethyl urocanate, ethyl urocanate ester, 4-tert-butyl-4'-methoxybenzoylmethane, 2-(2'-hydroxy-5'-methylphenyl) benzotriazole, 2-phenyl-5-methylbenzoxazole, methyl anthranilate and 2-Ethylhexyl dimethoxybenzylidene dioxoimidazolidine propionate.
The ultraviolet absorbers may be used alone or in combination.

"Moisturizing agents" refer to any moisturizing agents that are commonly used for mixing with the resin for cosmetics, and there is no particular limitation.
Examples of moisturizing agents include sorbitol, xylitol, propylene glycol, dipropylene glycol, 1,3-butylene glycol, glycerol, diglycerol, polyethylene glycol, hyaluronic acid, chondroitin sulfuric acid, pyrrolidone carboxylate and DL-pyrrolidone carboxylate.
The moisturizing agents may be used alone or in combination.

"Antiseptics" refer to any antiseptics that are commonly used for mixing with the resin for cosmetics, and there is no particular limitation.
Examples of antiseptics include p-oxybenzoic acid alkyl ester, benzoic acid, sodium benzoate, sorbic acid, potassium sorbate, phenoxyethanol, dehydroacetic acid and their salts.
The antiseptics may be used alone or in combination.

"Antibacterial agents" refer to any antibacterial agents that are commonly used for mixing with the resin for cosmetics, and there is no particular limitation.
Examples of antibacterial agents include benzoic acid, salicylic acid, carbolic acid, sorbic acid, p-oxybenzoic acid ester, parachlorometacresol, hexachlorophene, benzalkonium chloride, chlorhexidine chloride, trichlorocarbanilide, photosensitive agents and phenoxyethanol.
The antibacterial agents may be used alone or in combination.

"Antioxidants" refer to any antioxidants that are commonly used for mixing with the resin for cosmetics, and there is no particular limitation.
Examples of antioxidants include tocopherol, butylhydroxyanisole and dibutylhydroxytoluene.
The antioxidants may be used alone or in combination.

"pH modifiers" refer to any pH modifiers that are commonly used for mixing with the resin for cosmetics, and there is no particular limitation.
Examples of pH modifiers include lactic acid, citric acid, glycolic acid, succinic acid, tartaric acid, dl-malic acid, potassium carbonate, sodium hydrogen carbonate and ammonium hydrogen carbonate.
The pH modifiers may be used alone or in combination.

"Chelating agents" refer to any chelating agents that are commonly used for mixing with the resin for cosmetics, and there is no particular limitation.
Examples of chelating agents include alanine, sodium edetate, sodium polyphosphate, sodium metaphosphate and phosphoric acid.
The chelating agents may be used alone or in combination.

"Refrigerants" refer to any refrigerants that are commonly used for mixing with the resin for cosmetics, and there is no particular limitation.
Examples of refrigerants include L-menthol and camphor.
The refrigerants may be used alone or in combination.

"Antiinflammatory agents" refer to any antiinflammatory agents that are commonly used for mixing with the resin for cosmetics, and there is no particular limitation.
Examples of antiinflammatory agents include allantoin, glycyrrhetinic acid, tranexamic acid and azulene.
The antiinflammatory agents may be used alone or in combination.

A resin composition for cosmetic and a cosmetic of the present invention may be prepared in any forms that are commonly used for cosmetics, and there is no particular limitation. For example, a resin composition for cosmetic and a cosmetic according to the present invention can be used in a variety of forms, including solution, dispersion, cream, O/W or W/O emulsion such as milky lotion, paste, gel, aerosol and multilayer separate liquid.

It is possible to obtain cosmetics by using the above-described resin composition for cosmetics of the present invention as one component and mixing or reacting this composition with other components. "Other components" mentioned herein are not limited to the previously described various additives, and include other compounds (e.g., other polymer emulsions) that can serve as the main components of cosmetics.

As the method for "mixing" with other components, any commonly used mixing methods may be employed, and there is no particular limitation. Examples of such mixing methods include methods using mixers such as a ball mill, a homogenizer and a stirrer with blades.

More specifically, a resin for cosmetics of the present invention is first added to a suitable medium, preferably, an aqueous medium. Thereafter, it is possible to obtain a resin composition for cosmetics by adding various additives to the resin for cosmetics. Here, the order of mixing the resin for cosmetics and various additives may be appropriately selected, and there is no particular limitation. Mixers such as a ball mill, a homogenizer and a stirrer with blades may be used as necessary.

It is possible to obtain a cosmetic of the present invention by appropriately mixing the thus obtained resin composition for cosmetics with other suitable components. A cosmetic of the present invention can be used as, for example, make-up cosmetics, skin cosmetics and hair cosmetics by appropriately selecting other components that are mixed with the resin composition for cosmetics.

Examples of "make-up cosmetics" include eyeliners, eye shadows, eyebrow pencils, mascaras, manicures, liquid foundations, and lipsticks.
Examples of "skin cosmetics" include foundations, packs, cheek rouges, sunscreens, milky lotions, creams and skin lotions.
Examples of "hair cosmetics" include hair colorants, hair dyes, perming agents, bleaching agents, hair foams, hair creams, hair waxes, non aerosol pump sprays, hair gels, shampoos and rinses.

### Examples

Hereinafter, the present invention will be described more specifically and in detail by way of working examples and comparative examples. However, these working examples do not limit the present invention in any way.
The following monomers, emulsifiers and the like are used to produce the resins for cosmetics of the examples and the comparative examples:
(a1) methacrylic acid and (a2) acrylic acid as (A);
(b1) methyl methacrylate and (b2) butyl acrylate as (B);
(c1) 2-ethylhexyl-acrylate as (C);
(d1) ELEMINOL JS-2 (trademark) manufactured by Sanyo Chemical Industries, Ltd., which is a reactive emulsifier, and (d2) PE350 (trademark) manufactured by NOF CORPORATION, which is a reactive emulsifier; and
(e1) dodecyl mercaptan as (E).
In addition, (d3)' sodium lauryl sulfate (EMAL 0 (trademark) manufactured by Kao Corporation), which is a non-reactive emulsifier, is also used.

### Method for producing resins for cosmetics of Examples 1 to 15 and Comparative Examples 1 to 3 and 5 to 16

140 parts by weight of ion-exchange water and 0.1 parts by weight of a surfactant were added to a four-opening flask with a stirring blade, a thermometer and a reflux condenser. While blowing a nitrogen gas into the flask, the solution was heated and stirred, maintaining the temperature at 80°C. Meanwhile, a monomer mixture containing 30 parts by weight of ion-exchange water as well as surfactants, a chain transfer agent and monomers at the ratios shown in Tables 1 to 4, and an aqueous solution containing 0.3 parts by weight of potassium persulfate and 20 parts by weight of ion-exchange water were prepared. 5 wt% of the monomer mixture and 10 wt% of the above-described aqueous solution were added to the four-opening flask and stirred to initiate an emulsion polymerization reaction. Thereafter, the residue of the monomer mixture and the above-described aqueous solution were simultaneously added dropwise to the four-opening flask over about three hours. Further, the solution was continuously stirred for one hour while maintaining the temperature at 80°C, followed by cooling the resulting reaction mixture to 50°C. After adding ammonia water to the mixture to adjust the pH to about 8, the mixture was cooled to room temperature, thereby obtaining the target resins for cosmetics of Examples 1 to 15 and Comparative Examples 1 to 3 and 5 to 16.

### Method for producing resin for cosmetics of Comparative Example 4

70 parts by weight of isopropyl alcohol was added to a four-opening flask with a stirring blade, a thermometer and a reflux condenser. While blowing a nitrogen gas into the flask, the solution was heated and stirred, maintaining the temperature at 80°C. Meanwhile, a monomer mixture with the ratios shown in Table 3 and 0.3 parts by weight of 2,2-azobisisobutyronitrile were prepared. Thereafter, 2,2-azobisisobutyronitrile was added to the four-opening flask and stirred, and the monomer mixture was added dropwise to the four-opening flask over about three hours. Further, the solution was continuously stirred for one hour while maintaining the temperature at 80°C, followed by cooling the resulting reaction mixture to 40°C. After adding ammonia water to the mixture to adjust the pH to about 8, 170 parts by weight of water was added to the mixture, followed by cooling to room temperature. Then, isopropyl alcohol was removed from the mixture under reduced pressure, thereby obtaining the target resin for cosmetics of Comparative Example 4.

### Method for measuring weight average molecular weight

The weight average molecular weight was measured by using GPC (Waters 600E, 717, 410). As the columns, (Shodex KF-G, KF-804L, KF-803L) were used, and the temperature was 40°C. Additionally, THF was used as the eluent for the measurement.

### Measurement of transmittance

The transmittance was measured at a wavelength of 425 nm by using a spectrophotometer (U-3000 (trademark) manufactured by Hitachi, Ltd.). In addition, a diluted solution with a resin content of 0.6 wt% was used for the measurement of the transmittance.

### Transparency

The transparency was evaluated by measuring the transmittance by the predetermined method described above. The transparency was evaluated based on the following evaluation criteria:
"A" means that the transparency was 60% or more;
"B" means that the transparency was 50% or more and less than 60%;
"C" means that the transparency was 30% or more and less than 50%; and
"D" means that the transparency was less than 30%.

### Water resistance

The water dispersions of the examples and the comparative examples were applied onto glass plates using a 10 ml applicator, and dried for 12 hours at 50°C. Then, the resulting films were placed in warm water at a temperature of 30°C, and the states of the films were evaluated after 30 minutes by visual observation. The water resistance of each film was evaluated based on the following evaluation criteria:
"A" means that no change was observed in the film from the dried state at all;
"B" means that swelling of the film could be observed, but dissolution could not be observed;
"C" means that swelling and partial dissolution of the film could be observed; and
"D" means that an obvious dissolution of the film could be observed.

### Adhesion

The adhesion was evaluated by an actual application test by 10 test persons. The water dispersions of the examples and the comparative examples were diluted with distilled water to have a resin content of 5 wt%. 0.1 g of each of these solutions was applied around the eyes of the test persons, and the adhesion was evaluated. The adhesion was evaluated based on the following evaluation criteria:
"A" means that 8 to 10 test persons felt that the adhesion was good;
"B" means that 5 to 7 test persons felt that the adhesion was good;
"C" means that 2 to 4 test persons felt that the adhesion was good; and
"D" means that 0 to 1 test person felt that the adhesion was good.

### Lasting effect

The lasting effect was evaluated by an actual application test by 10 test persons. The water dispersions of the examples and the comparative examples were diluted with distilled water to have a resin content of 5 wt%. 0.1 g of each of these solutions was applied around the eyes of the test persons, and the lasting effect was evaluated. The lasting effect was evaluated based on the following evaluation criteria:
"A" means that 8 to 10 test persons felt that the lasting effect was good;
"B" means that 5 to 7 test persons felt that the lasting effect was good;
"C" means that 2 to 4 test persons felt that the lasting effect was good; and
"D" means that 0 to 1 test person felt that the lasting effect was good.

### Pulling feel

The pulling feel was evaluated by an actual application test by 10 test persons. The water dispersions of the examples and the comparative examples were diluted with distilled water to have a resin content of 5 wt%. 0.1 g of each of these solutions was applied around the eyes of the test persons, and the pulling feel was evaluated. The pulling feel was evaluated based on the following evaluation criteria:
"A" means that 8 to 10 test persons had a natural feeling without any pulling feel;
"B" means that 5 to 7 test persons had a natural feeling without any pulling feel;
"C" means that 2 to 4 test persons had a natural feeling without any pulling feel; and
"D" means that 0 to 1 test person had a natural feeling without any pulling feel.

**Table 1**

| | Example | | | | | | | |
|---|---|---|---|---|---|---|---|---|
| | 1 | 2 | 3 | 4 | 5 | 6 | 7 | 8 |
| Composition | | | | | | | | |
| (A) (a1) | 14.0 | | 10.0 | 20.0 | 20.0 | 10.0 | 15.0 | 15.0 |
| (a2) | | 14.0 | | | | | | |
| (B) (b1) | 41.0 | 41.0 | 43.0 | 40.0 | 30.0 | 40.0 | 45.0 | 25.0 |
| (b2) | 20.0 | 20.0 | 22.0 | 20.0 | 10.0 | 40.0 | 30.0 | 20.0 |
| (C) (c1 ) | 25.0 | 25.0 | 25.0 | 20.0 | 40.0 | 10.0 | 10.0 | 40.0 |
| (D) (d1) | 0.6 | 0.6 | 0.6 | 0.6 | 0.6 | 0.6 | 0.6 | 0.6 |
| (d2) | | | | | | | | |
| (d3)' | | | | | | | | |
| (E) (e1) | 1.0 | 1.0 | 1.0 | 1.0 | 1.0 | 1.0 | 1.0 | 1.0 |
| Molecular weight¹⁾ | 44000 | 43000 | 45000 | 45000 | 44000 | 43000 | 45000 | 45000 |
| Evaluation | | | | | | | | |
| Transparency | A | B | B | A | B | B | A | B |
| Water resistance | A | B | B | B | A | B | B | A |
| Adhesion | A | A | A | A | B | A | A | B |
| Lasting effect | A | A | A | B | B | B | B | B |
| Pulling feel | A | A | A | B | A | B | B | A |

| | | | | | | | | |
|---|---|---|---|---|---|---|---|---|
| 1) The molecular weight is a weight average molecular weight. | | | | | | | | |

**Table 2**

| | Example | | | | | | |
|---|---|---|---|---|---|---|---|
| | 9 | 10 | 11 | 12 | 13 | 14 | 15 |
| Composition | | | | | | | |
| (A) (a1) | 14.0 | 14.0 | 14.0 | 14.0 | 14.0 | 14.0 | 14.0 |
| (a2) | | | | | | | |
| (B) (b1) | 41.0 | 41.0 | 41.0 | 41.0 | 41.0 | 41.0 | 41.0 |
| (b2) | 20.0 | 20.0 | 20.0 | 20.0 | 20.0 | 20.0 | 20.0 |
| (C) (c1 ) | 25.0 | 25.0 | 25.0 | 25.0 | 25.0 | 25.0 | 25.0 |
| (D) (d1) | | 0.6 | 0.6 | 0.6 | | 1.8 | 2.5 |
| (d2) | 0.6 | | | | | | |
| (d3)' | | | | | 0.6 | | |
| (E) (e1) | 1.0 | 2.0 | 0.5 | | 1.0 | 1.0 | 1.0 |
| Molecular weight¹⁾ | 46000 | 15000 | 75000 | 100000 | 45000 | 45000 | 43000 |
| Evaluation | | | | | | | |
| Transparency | A | A | A | B | B | A | A |
| Water resistance | A | B | A | A | B | A | B |
| Adhesion | A | A | A | A | B | A | A |
| Lasting effect | A | A | A | A | B | A | A |
| Pulling feel | A | A | A | A | B | A | A |

| | | | | | | | |
|---|---|---|---|---|---|---|---|
| 1) The molecular weight is a weight average molecular weight. | | | | | | | |

**Table 3**

| | Comparative Example | | | | | | | |
|---|---|---|---|---|---|---|---|---|
| | 1 | 2 | 3 | 4 | 5 | 6 | 7 | 8 |
| Composition | | | | | | | | |
| (A) (a1) | | 10.0 | 10.0 | 14.0 | 9.0 | 7.0 | 21.0 | 23.0 |
| (a2) | | | | | | | | |
| (B) (b1) | 40.0 | | 45.0 | 41.0 | 45.0 | 45.0 | 34.0 | 32.0 |
| (b2) | 30.0 | | 45.0 | 20.0 | 21.0 | 23.0 | 20.0 | 20.0 |
| (C) (c1) | 30.0 | 90.0 | | 25.0 | 25.0 | 25.0 | 25.0 | 25.0 |
| (D) (d1) | 0.6 | 0.6 | 0.6 | | | | | |
| (d2) | | | | | | | | |
| (d3)' | | | | | 0.6 | 0.6 | 0.6 | 0.6 |
| (E) (e1) | 1.0 | 1.0 | 1.0 | 1.0 | 1.0 | 1.0 | 1.0 | 1.0 |
| Molecular weight¹⁾ | 48000 | 47000 | 45000 | 9000 | 45000 | 44000 | 45000 | 47000 |
| Evaluation | | | | | | | | |
| Transparency | D | D | B | B | C | D | B | B |
| Water resistance | B | B | D | D | B | B | C | D |
| Adhesion | D | D | D | C | D | D | C | D |
| Lasting effect | D | D | D | D | D | D | D | D |
| Pulling feel | D | B | D | B | D | D | D | D |

| | | | | | | | | |
|---|---|---|---|---|---|---|---|---|
| 1) The molecular weight is a weight average molecular weight. | | | | | | | | |

**Table 4**

| | Comparative Example | | | | | | | |
|---|---|---|---|---|---|---|---|---|
| | 9 | 10 | 11 | 12 | 13 | 14 | 15 | 16 |
| Composition | | | | | | | | |
| (A) (a1) | 20.0 | 20.0 | 10.0 | 10.0 | 15.0 | 15.0 | 15.0 | 15.0 |
| (a2) | | | | | | | | |
| (B) (b1) | 29.0 | 25.0 | 41.0 | 45.0 | 46.0 | 48.0 | 24.0 | 22.0 |
| (b2) | 10.0 | 10.0 | 40.0 | 40.0 | 30.0 | 30.0 | 20.0 | 20.0 |
| (C) (c1) | 41.0 | 45.0 | 9.0 | 5.0 | 9.0 | 7.0 | 41.0 | 43.0 |
| (D) (d1) | | | | | | | | |
| (d2) | | | | | | | | |
| (d3)' | 0.6 | 0.6 | 0.6 | 0.6 | 0.6 | 0.6 | 0.6 | 0.6 |
| (E) (e1) | 1.0 | 1.0 | 1.0 | 1.0 | 1.0 | 1.0 | 1.0 | 1.0 |
| Molecular weight¹⁾ | 45000 | 47000 | 43000 | 45000 | 45000 | 44000 | 46000 | 45000 |
| Evaluation | | | | | | | | |
| Transparency | C | D | D | C | B | B | C | D |
| Water resistance | D | C | C | D | C | D | C | D |
| Adhesion | C | D | C | D | C | D | C | D |
| Lasting effect | D | D | D | D | D | D | D | D |
| Pulling feel | D | D | D | D | D | D | D | D |

| | | | | | | | | |
|---|---|---|---|---|---|---|---|---|
| 1) The molecular weight is a weight average molecular weight. | | | | | | | | |

Each of the resins for cosmetics of Examples 1 to 15 yields excellent balance between properties such as water resistance, adhesion, lasting effect and pulling feel despite having superior transparency, and is generally excellent as a film-forming agent for cosmetics. On the other hand, Comparative Examples 1 to 16 are inferior in the balance between properties such as water resistance, adhesion, lasting effect and pulling feel, although having superior transparency. It should be noted that Comparative Example 4 is a resin for cosmetics that is obtained by solution polymerization. Comparing Comparative Example 4 with Example 1, it can be seen that it is important to produce the resin for cosmetics by emulsion polymerization.

The above-described resin for cosmetics of the present invention can be used for a variety of resin compositions for cosmetics and cosmetics. Examples of the cosmetics containing these resin compositions for cosmetics are shown below.

As examples of the make-up cosmetics, mascaras, eyeliners, manicures, liquid eye shadows and liquid foundations were produced.
As for the mascaras, the components from stearic acid to liquid paraffin listed in Table 5 were mixed at the ratios shown in Table 5, and the mixture was dissolved at 80°C, preparing an oil phase. Next, the components from propylene glycol to water were mixed at the ratios shown in Table 5, and the mixture was dissolved at 80°C, followed by dispersing a pigment in the mixture to prepare a water phase. The oil phase was charged into the water phase, and the mixture was emulsified with a homomixer. Then, the components from the resin for cosmetics of Example 1 to octylacrylamide/acrylate copolymer were gradually added to the mixture at the ratio shown in Table 5, and the whole was stirred with a homomixer. Thereafter, the mixture was cooled to room temperature, thereby producing a mascara.

**Table 5**

| Mascara | Example | Comparative Example |
|---|---|---|
| Composition | 21 | 21 |
| Stearic acid | 5.0 | 5.0 |
| Beeswax | 6.0 | 6.0 |
| Liquid paraffin | 3.0 | 3.0 |
| Propylene glycol | 5.0 | 5.0 |
| Bentonite | 2.0 | 2.0 |
| Sodium lauryl sulfate | 0.5 | 0.5 |
| Triethanolamine | Proper amount | Proper amount |
| Antiseptic | Proper amount | Proper amount |
| Water | Remainder | Remainder |
| Pigment | 10.0 | 10.0 |
| Resin for cosmetics of Ex.1 | 10.0 | |
| Octylacrylamide/acrylate copolymer | 5.0 | 5.0 |
| Total | 100 | 100 |

As for the eyeliners, the components from polyoxyethylene sorbitan monostearate to xanthan gum listed in Table 6 were mixed at the ratios shown in Table 6, and the mixture was uniformly dispersed with a colloid mill. To this dispersion, the components from glycerol to the resin for cosmetics of Example 1 were successively mixed at the ratios shown in Table 6, and the whole was stirred until it became homogeneous, thereby producing an eyeliner.

**Table 6**

| Eyeliner | Example | Comparative Example |
|---|---|---|
| Composition | 22 | 22 |
| Polyoxyethylene sorbitan monostearate | 1.0 | 1.0 |
| Titanium dioxide | 2.0 | 2.0 |
| Iron oxide | 5.0 | 5.0 |
| Triethanolamine | Proper amount | Proper amount |
| Antiseptic | Proper amount | Proper amount |
| Water | Remainder | Remainder |
| xanthan gum | 1.0 | 1.0 |
| Glycerol | 5.0 | 5.0 |
| Acetyl tributyl citrate | 1.0 | 1.0 |
| Resin for cosmetics of Ex. 1 | 10.0 | |
| Total | 100 | 100 |

As for the manicures, the components from polyoxyethylene sorbitan monooleate to water listed in Table 7 were mixed at the ratios shown in Table 7, and the mixture was stirred until it became homogeneous. To this mixture, a pigment was added and dispersed at the ratio shown in Table 7, and the components from (acrylates/steareth-20 itaconate) copolymer to an acrylic resin emulsion were successively added at the ratio shown in Table 7, followed by stirring until it became homogeneous, thereby producing a manicure.

**Table 7**

| Manicure | Example | Comparative Example |
|---|---|---|
| Composition | 23 | 23 |
| Polyoxyethylene sorbitan monooleate | 1.0 | 1.0 |
| Carbitol | 5.0 | 5.0 |
| Diethyl phthalate | 4.0 | 4.0 |
| Triethanolamine | Proper amount | Proper amount |
| Antiseptic | Proper amount | Proper amount |
| Water | Remainder | Remainder |
| Pigment | 2.0 | 2.0 |
| (acrylates/steareth-20 itaconate) copolymer | 0.5 | 0.5 |
| Resin for cosmetics of Ex. 1 | 10.0 | |
| Acrylic resin emulsion (resin content 45%) | 30.0 | 30.0 |
| Total | 100 | 100 |

As for the liquid eye shadows, the components from microcrystalline wax to sorbitan monostearate listed in Table 8 were mixed at the ratios shown in Table 8, and the mixture was dissolved at 80°C, preparing an oil phase. Next, the components from dehydroxanthan gum to water were mixed at the ratios shown in Table 8, and the mixture was dissolved at 80°C, followed by dispersing a pigment in the mixture to prepare a water phase. The oil phase was charged into the water phase, and the mixture was emulsified with a homomixer. Then, the resin for cosmetics of Example 1 was gradually added to the mixture at the ratio shown in Table 8, and the whole was stirred with a homomixer. Thereafter, the mixture was cooled to room temperature, thereby producing a liquid eye shadow.

**Table 8**

| Liquid eye shadow | Example | Comparative Example |
|---|---|---|
| Composition | 24 | 24 |
| Microcrystalline wax | 3.0 | 3.0 |
| Stearic acid | 3.0 | 3.0 |
| Liquid paraffin | 10.0 | 10.0 |
| Lanolin | 1.0 | 1.0 |
| Sorbitan monostearate | 2.0 | 2.0 |
| dehydroxanthan gum | 0.5 | 0.5 |
| Glycerol | 4.0 | 4.0 |
| Triethanolamine | Proper amount | Proper amount |
| Antiseptic | Proper amount | Proper amount |
| Water | Remainder | Remainder |
| Pigment | 2.0 | 2.0 |
| Resin for cosmetics of Ex. 1 | 5.0 | |
| Total | 100 | 100 |

As for the liquid foundations, the components from the resin for cosmetics of Example 1 to water listed in Table 9 were mixed at the ratios shown in Table 9, and the mixture was dispersed at 70°C with a homomixer until it became homogeneous. Next, the components from talc to iron oxide were sufficiently mixed at the ratios shown in Table 9, and the mixture was added to the above-described dispersion. The whole was dispersed at 70°C with a homomixer to prepare a water phase. The components from stearic acid to liquid paraffin were mixed at the ratios shown in Table 9, and the mixture was dissolved at 80°C, preparing an oil phase. This oil phase was charged into the above-described water phase, and the mixture was emulsified at 70°C with a homomixer. Thereafter, the mixture was cooled to room temperature, thereby producing a liquid foundation.

**Table 9**

| Liquid foundation | Example | Comparative Example |
|---|---|---|
| Composition | 25 | 25 |
| Resin for cosmetics of Ex. 1 | 5.0 | |
| Bentonite | 0.5 | 0.5 |
| polyoxyethylene sorbitan monostearate | 1.0 | 1.0 |
| Propylene glycol | 10.0 | 10.0 |
| Triethanolamine | Proper amount | Proper amount |
| Antiseptic | Proper amount | Proper amount |
| Water | Remainder | Remainder |
| Talc | 3.0 | 3.0 |
| Titanium dioxide | 5.0 | 5.0 |
| Red oxide | 0.5 | 0.5 |
| Iron oxide | 1.0 | 1.0 |
| Stearic acid | 2.5 | 2.5 |
| Isohexadecyl alcohol | 7.0 | 7.0 |
| Glycerol monostearate | 2.0 | 2.0 |
| Liquid lanolin | 2.0 | 2.0 |
| Liquid paraffin | 8.0 | 8.0 |
| Total | 100 | 100 |

As examples of the skin cosmetics, O/W skin creams, W/O skin creams and milky lotions were produced.
As for the O/W skin creams, the components from propylene glycol to water (i.e., the components other than the components from C12-15 alkyl benzoate to an antiseptic) listed in Table 10 were mixed at the ratios shown in Table 10, and the mixture was dissolved at 80°C, preparing a water phase. Next, the components from C12-15 alkyl benzoate to an antiseptic were mixed at the ratios shown in Table 10, and the mixture was dissolved at 80°C, preparing an oil phase. The oil phase was charged into the water phase, and the mixture was emulsified with a homomixer, followed by stirring until it became homogeneous. Thereafter, the mixture was cooled to room temperature, thereby producing an O/W skin cream.

**Table 10**

| O/W skin cream | Example | Comparative Example |
|---|---|---|
| Composition | 26 | 26 |
| Propylene glycol | 3.0 | 3.0 |
| Triethanolamine | 0.5 | 0.5 |
| Resin for cosmetics of Ex.1 | 3.0 | |
| Water | Remainder | Remainder |
| C12-15 alkyl benzoate | 5.0 | 5.0 |
| Octyl palmitate | 5.0 | 5.0 |
| Cetyl alcohol | 1.0 | 1.0 |
| Stearic acid | 2.0 | 2.0 |
| Dimethicone polyol | 1.0 | 1.0 |
| Antiseptic | Proper amount | Proper amount |
| Total | 100 | 100 |

As for the W/O skin creams, the components from propylene glycol to water (i.e., the components other than the components from microcrystalline wax to an antiseptic) listed in Table 11 were mixed at the ratios shown in Table 11, and the mixture was dissolved at 70°C, preparing a water phase. Next, the components from microcrystalline wax to an antiseptic were mixed at the ratios shown in Table 11, and the mixture was dissolved at 70°C, preparing an oil phase. The oil phase was charged into the water phase, and the mixture was emulsified with a homomixer, followed by stirring until it became homogeneous. Thereafter, the mixture was cooled to room temperature, thereby producing a W/O skin cream.

**Table 11**

| W/O skin cream | Example | Comparative Example |
|---|---|---|
| Composition | 27 | 27 |
| Propylene glycol | 5.0 | 5.0 |
| Resin for cosmetics of Ex. 1 | 3.0 | |
| Water | Remainder | Remainder |
| Microcrystalline wax | 9.0 | 9.0 |
| Solid paraffin | 2.0 | 2.0 |
| Beeswax | 3.0 | 3.0 |
| Vaseline | 5.0 | 5.0 |
| Reduced lanolin | 5.0 | 5.0 |
| Squalane | 34.0 | 34.0 |
| Hexadecyl adipate | 10.0 | 10.0 |
| Glycerol monooleate | 3.5 | 3.5 |
| POE(25) sorbitan monooleate | 1.0 | 1.0 |
| Antiseptic | Proper amount | Proper amount |
| Total | 100 | 100 |

As for the milky lotions, the components from dipropylene glycol to water (i.e., the components other than the components from stearic acid to sorbitan monooleate) listed in Table 12 were mixed at the ratios shown in Table 12, and the mixture was dissolved at 70°C, preparing a water phase. Next, the components from stearic acid to sorbitan monooleate were mixed at the ratios shown in Table 12, and the mixture was dissolved at 70°C, preparing an oil phase. The oil phase was charged into the water phase, and the mixture was emulsified with a homomixer, followed by stirring until it became homogeneous. Thereafter, the mixture was cooled to room temperature, thereby producing a milky lotion.

**Table 12**

| Milky lotion | Example | Comparative Example |
|---|---|---|
| Composition | 28 | 28 |
| Dipropylene glycol | 5.0 | 5.0 |
| Resin for cosmetics of Ex. 1 | 1.0 | |
| Triethanolamine | Proper amount | Proper amount |
| Antiseptic | Proper amount | Proper amount |
| Water | Remainder | Remainder |
| Stearic acid | 2.0 | 2.0 |
| Cetyl alcohol | 1.5 | 1.5 |
| Vaseline | 4.0 | 4.0 |
| Squalane | 5.0 | 5.0 |
| Glyceryl tri-2-ethylhexanoate | 2.0 | 2.0 |
| Sorbitan monooleate | 2.0 | 2.0 |
| Total | 100 | 100 |

As examples of the hair cosmetics, shampoos, hair creams and hair colorants were produced.
As for the shampoos, the ingredients listed in Table 13 were mixed at the ratios shown in Table 13, and the mixture was dissolved at 80°C, followed by stirring until it became homogeneous. Thereafter, the mixture was cooled to room temperature, thereby producing a shampoo.

**Table 13**

| Shampoo | Example | Comparative Example |
|---|---|---|
| Composition | 29 | 29 |
| Sodium laureth sulfate | 10.0 | 10.0 |
| Cocamidopropyl betaine | 5.0 | 5.0 |
| Cocamido MEA | 2.0 | 2.0 |
| Lauramidopropyl betaine | 1.0 | 1.0 |
| Glycol distearate | 2.0 | 2.0 |
| Dimethicone | 1.0 | 1.0 |
| Dimethicone polyol | 1.0 | 1.0 |
| Potassium cocoyi glutamate | 0.5 | 0.5 |
| Laureth-4 | 1.0 | 1.0 |
| Hydrolyzed collagen | 0.2 | 0.2 |
| Panthenol | 0.2 | 0.2 |
| Citric acid | 0.1 | 0.1 |
| Antiseptic | Proper amount | Proper amount |
| Resin for cosmetics of Ex. 1 | 1.0 | |
| Water | Remainder | Remainder |
| Total | 100 | 100 |

As for the hair creams, the components from liquid paraffin to an antiseptic (i.e., the components other than the components from the resin for cosmetics of Example 1 to water) listed in Table 14 were mixed at the ratios shown in Table 14, and the mixture was dissolved at 80°C, preparing an oil phase. Next, the components from the resin for cosmetics of Example 1 to water were mixed at the ratios shown in Table 14, and the mixture was dissolved at 80°C, preparing a water phase. The oil phase was charged into the water phase, and the mixture was emulsified with a homomixer, followed by stirring until it became homogeneous. Thereafter, the mixture was cooled to room temperature, thereby producing a hair cream.

**Table 14**

| Hair cream | Example | Comparative Example |
|---|---|---|
| Composition | 30 | 30 |
| Liquid paraffin | 15.0 | 15.0 |
| Vaseline | 15.0 | 15.0 |
| Beeswax | 3.0 | 3.0 |
| Sorbitan stearate | 3.0 | 3.0 |
| Polyoxyethylene cetyl ether | 2.0 | 2.0 |
| Glycerol | 5.0 | 5.0 |
| Dimethicone | 1.0 | 1.0 |
| Triethanolamine | Proper amount | Proper amount |
| Antiseptic | Proper amount | Proper amount |
| Resin for cosmetics of Ex. 1 | 3.0 | |
| Water | Remainder | Remainder |
| Total | 100 | 100 |

As for the hair colorants, the components from benzyl alcohol to the resin for cosmetics of Example 1 (i.e., the components other than a dye) listed in Table 15 were mixed at the ratios shown in Table 15, and the mixture was dissolved at 80°C. Next, a dye was mixed at the ratios shown in Table 15. The mixture was emulsified with a homomixer, and stirred until it became homogeneous. Then, the mixture was cooled to room temperature, thereby producing a hair colorant.

**Table 15**

| Hair colorant | Example | Comparative Example |
|---|---|---|
| Composition | 31 | 31 |
| Benzyl alcohol | 10.0 | 10.0 |
| Citric acid | 1.5 | 1.5 |
| Hydroxyethyl cellulose | 0.5 | 0.5 |
| Water | Remainder | Remainder |
| Resin for cosmetics of Ex 1 | 10.0 | |
| dye | 0.2 | 0.2 |
| Total | 100 | 100 |

The resin for cosmetics of the present invention is a resin for cosmetics that is obtained by emulsion polymerization of a monomer mixture containing:
(A) component: at least one selected from the group consisting of acrylic acid, methacrylic acid, maleic acid, itaconic acid and crotonic acid,
(B) component: at least one selected from the group consisting of alkyl(meth)acrylate monomers having an alkyl group with a carbon number of 1 to 4 and
(C) component: at least one selected from the group consisting of alkyl(meth)acrylate monomers having an alkyl group with a carbon number of 5 or more,
wherein the monomer mixture contains 10 to 20 parts by weight of the (A) component, 40 to 80 parts by weight of the (B) component and 10 to 40 parts by weight of the (C) component, based upon the total of the (A) to (C) components (100 parts by weight). Therefore, it is possible to provide a generally excellent resin for cosmetics as a film-forming agent for cosmetics that yields excellent balance between properties such as water resistance, adhesion, lasting effect and pulling feel despite having superior transparency.

Since the (A) component is methacrylic acid, the resin for cosmetics of the present invention has an improved transparency and a better water resistance than resins for cosmetics using acrylic acid.
Since a (D) reactive emulsifier is used at the time of the emulsion polymerization and the (D) reactive emulsifier is an anionic surfactant and/or nonionic surfactant, the residual hydrophilic low molecular weight materials can be decreased for the resin for cosmetics of the present invention. Therefore, it has an improved water resistance, less grids (aggregates), an increased yield and an improved polymerization degree of the (A) component.

Since the (D) reactive emulsifier is an reactive emulsifier that has a carboxyl group, a sulfonic acid group, a sulfonate group, a sulfate group or an ethylenoxy group, and further has an ethylenic double bond, the resin for cosmetics of the present invention has superior transparency, a further improved balance between the properties of water resistance, adhesion, lasting effect and pulling feel, as well as a further improved polymerization degree of the (A) component.
Since a (E) chain transfer agent is additionally used at the time of the emulsion polymerization, the resin for cosmetics of the present invention has a controlled molecular weight and a further improved transparency.
Since the weight average molecular weight is 1000 to 100000, the resin for cosmetics of the present invention has a further improved transparency and a further improved balance between the properties of water resistance, adhesion, lasting effect and pulling feel.
The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A resin for cosmetics obtained by emulsion polymerization of a monomer mixture containing:
(A) component at least one selected from the group consisting of acrylic acid, methacrylic acid, maleic acid, itaconic acid and crotonic acid,
(B) component: at least one selected from the group consisting of alkyl(meth)acrylate monomers having an alkyl group with a carbon number of 1 to 4, and
(C) component: at least one selected from the group consisting of alkyl(meth)acrylate monomers having an alkyl group with a carbon number of 5 or more,
wherein the monomer mixture contains 10 to 20 parts by weight of the (A) component, 40 to 80 parts by weight of the (B) component and 10 to 40 parts by weight of the (C) component, based upon the total of the (A) to (C) components (100 parts by weight).

2. The resin for cosmetics according to claim 1,
wherein the (A) component is methacrylic acid.

3. The resin for cosmetics according to claim 1 or 2,
wherein a (D) reactive emulsifier is used at the time of the emulsion polymerization and the (D) reactive emulsifier is an anionic surfactant and/or nonionic surfactant.

4. The resin for cosmetics according to claim 3,
wherein the (D) reactive emulsifier has a carboxyl group, a sulfonic acid group, a sulfonate group, a sulfate group or an ethylenoxy group, and further has an ethylenic double bond.

5. The resin for cosmetics according to any one of claims 1 to 4,
wherein a (E) chain transfer agent is further used at the time of the emulsion polymerization.

6. The resin for cosmetics according to any one of claims 1 to 5, having a weight average molecular weight of 1000 to 100000.

7. The resin for cosmetics according to any one of claims 1 to 6,
having a transmittance of light with a wavelength of 425 nm of at least 50%.

8. A resin composition for cosmetics comprising the resin for cosmetics according to claims 1 to 7 as a main component.

9. A cosmetic in which the resin for cosmetics according to claim 8 is mixed as one component.

10. The cosmetic according to claim 9 that is used as a make-up cosmetic, a skin cosmetic and a hair cosmetic.

## Patentansprüche

1. Harz für Kosmetika, das durch Emulsionspolymerisation eines Monomerengemisches erhalten wird, welches enthält:
Komponente (A): wenigstens eines, ausgewählt aus der Gruppe bestehend aus Acrylsäure, Methacrylsäure, Maleinsäure, Itaconsäure und Crotonsäure;
Komponente (B): wenigstens eines, ausgewählt aus der Gruppe bestehend aus Alkyl(meth)acrylat-Monomeren, die eine Alkylgruppe mit einer Kohlenstoffzahl von 1 bis 4 haben, und
Komponente (C): wenigstens eines, ausgewählt aus der Gruppe bestehend aus Alkyl(meth)acrylat-Monomeren, die eine Alkylgruppe mit einer Kohlenstoffzahl von 5 oder mehr haben;
wobei das Monomerengemisch 10 bis 20 Gew.-Teile der Komponente (A), 40 bis 80 Gew.-Teile der Komponente (B) und 10 bis 40 Gew.-Teile der Komponente (C), bezogen auf die Gesamtmenge der Komponenten (A) bis (C) (100 Gew.-Teile), enthält.

2. Harz für Kosmetika nach Anspruch 1, wobei die Komponente (A) Methacrylsäure ist.

3. Harz für Kosmetika nach Anspruch 1 oder 2, wobei ein reaktiver Emulgator (D) zur Zeit der Emulsionspolymerisation verwendet wird und der reaktive Emulgator (D) ein anionisches, oberflächenaktives Mittel und/oder ein nicht-ionisches, oberflächenaktives Mittel ist.

4. Harz für Kosmetika nach Anspruch 3, wobei der reaktive Emulgator (D) eine Carboxylgruppe, eine Sulfonsäuregruppe, eine Sulfonatgruppe, eine Sulfatgruppe oder eine Ethylenoxygruppe hat, und außerdem eine ethylenische Doppelbindung hat.

5. Harz für Kosmetika nach einem der Ansprüche 1 bis 4, wobei außerdem ein Kettenübertragungsmittel (E) zur Zeit der Emulsionspolymerisation verwendet wird.

6. Harz für Kosmetika nach einem der Ansprüche 1 bis 5, das ein gewichtsmittleres Molekulargewicht von 1000 bis 100000 hat.

7. Harz für Kosmetika nach einem der Ansprüche 1 bis 6, das einen Transmissionsgrad für Licht mit einer Wellenlänge von 425 nm von wenigstens 50 % hat.

8. Harzzusammensetzung für Kosmetika, die das Harz für Kosmetika nach den Ansprüchen 1 bis 7 als Hauptkomponente umfasst.

9. Kosmetikum, in dem das Harz für Kosmetika nach Anspruch 8 als eine Komponente eingemischt ist.

10. Kosmetikum nach Anspruch 9, das als ein Make-up-Kosmetikum, ein Haut-Kosmetikum und ein Haar-Kosmetikum verwendet wird.

## Revendications

1. Résine pour produits cosmétiques obtenue par polymérisation en émulsion d'un mélange de monomères contenant :
constituant (A) : au moins un constituant choisi dans le groupe consistant en l'acide acrylique, l'acide méthacrylique, l'acide maléique, l'acide itaconique et l'acide crotonique,
constituant (B) : au moins un constituant choisi dans le groupe consistant en des monomères (méth)acrylate d'alkyle ayant un groupe alkyle de 1 à 4 atomes de carbone, et
constituant (C) : au moins un constituant choisi dans le groupe consistant en des monomères (méth)acrylate d'alkyle ayant un groupe alkyle de 5 ou plus de 5 atomes de carbone,
dans laquelle le mélange de monomères contient 10 à 20 parties en poids du constituant (A), 40 à 80 parties en poids du constituant (B) et 10 à 40 parties en poids du constituant (C), sur la base du total des constituants (A) à (C) (100 parties en poids).

2. Résine pour produits cosmétiques suivant la revendication 1, dans laquelle le constituant (A) est l'acide méthacrylique.

3. Résine pour produits cosmétiques suivant la revendication 1 ou 2, dans laquelle un émulsionnant réactif (D) est utilisé lors de la polymérisation en émulsion et l'émulsionnant réactif (D) est un agent tensioactif anionique et/ou un agent tensioactif non ionique.

4. Résine pour produits cosmétiques suivant la revendication 3, dans laquelle l'émulsionnant réactif (D) possède un groupe carboxyle, un groupe acide sulfonique, un groupe sulfonate, un groupe sulfate ou un groupe éthylènoxy et possède en outre une double liaison éthylénique.

5. Résine pour produits cosmétiques suivant l'une quelconque des revendications 1 à 4, dans laquelle un agent de transfert de chaîne (E) est en outre utilisé lors de la polymérisation en émulsion.

6. Résine pour produits cosmétiques suivant l'une quelconque des revendications 1 à 5, ayant une moyenne en poids du poids moléculaire de 1000 à 100 000.

7. Résine pour produits cosmétiques suivant l'une quelconque des revendications 1 à 6, ayant une transmittance de lumière à une longueur d'onde de 425 nm d'au moins 50 %.

8. Composition de résine pour produits cosmétiques, comprenant la résine pour produits cosmétiques suivant les revendications 1 à 7 comme constituant principal.

9. Produit cosmétique dans lequel la résine pour produits cosmétiques suivant la revendication 8 est mélangée comme constituant.

10. Produit cosmétique suivant la revendication 9, qui est utilisé comme produit cosmétique de maquillage, produit cosmétique pour la peau ou produit cosmétique capillaire.
